# EUROPÄISCHE PATENTSCHRIFT

(11) **EP 2 816 096 B1**
(45) Veröffentlichungstag und Bekanntmachung des Hinweises auf die Patenterteilung: **12.05.2021**
(21) Anmeldenummer: 13172441.1
(22) Anmeldetag: 18.06.2013
(51) Int. Cl.: C10K 3/00

(54) **VERFAHREN UND VORRICHTUNG ZUR SPEICHERUNG VON ÜBERSCHUSSENERGIE**
METHOD AND APPARATUS FOR STORING EXCESS ENERGY
PROCÉDÉ ET DISPOSITIF DE STOCKAGE D'ÉNERGIE EN EXCÈS

(43) Veröffentlichungstag der Anmeldung: 24.12.2014
(73) Patentinhaber: Evonik Operations GmbH, 45128 Essen (DE)
(72) Erfinder: Haas, Thomas, 48161 Münster (DE)
(74) Vertreter: Evonik Patent Association

(56) Entgegenhaltungen:
- WO-A1-2007/123510
- WO-A1-2012/026833
- WO-A2-2008/028055
- WO-A2-2009/154788
- WO-A2-2012/151545
- WO-A2-2012/174313
- US-A1- 2010 132 257
- US-A1- 2011 067 376
- JACQUELINE L COTTER ET AL: "Ethanol and acetate production by Clostridium ljungdahlii and Clostridium autoethanogenum using resting cells", BIOPROCESS AND BIOSYSTEMS ENGINEERING, SPRINGER, DE, vol. 32, no. 3, 1 April 2009 (2009-04-01), pages 369-380, XP002671948, ISSN: 1615-7591, DOI: 10.1007/S00449-008-0256-Y [retrieved on 2008-08-26]
- A. Kargari, M.T. Ravanchi: "Carbon Dioxide: capturing and Utilization" In: "Greenhouse Gases - capturing, Utilization and Reduction", 9 March 2012 (2012-03-09), InTech ISBN: 978-953-51-0192-5 pages 1-30,

## Beschreibung

### Gebiet der Erfindung

Gegenstand der Erfindung ist ein Verfahren und eine Vorrichtung zur Verwertung von Gasen enthaltend CO und/oder CO₂ wie in den Ansprüchen offenbart.

### Stand der Technik

Stromproduzierende Kraftwerke produzieren bei mangelnder Abnahme überschüssigen Strom. Dieser muss entsprechend in anderer Form gespeichert werden.

So werden z.B. Pumpspeicher gebaut, um Überschusselektrizität zu speichern. Pumpspeicher haben zwar eine große Speicherkapazität aber auch einen großen Raum und Flächenbedarf und stellen nicht unerhebliche Eingriffe in Ökosysteme und Landschaft dar.

Ein weiterer Ansatz ist es elektrische Energie in großen Batterien, insbesondere Lithiumionenbatterien zu speichern. Diese Technologie erfordert jedoch sehr hohe Investitionen in zusätzliche Batterien, deren Abschreibung den Vorteil Nutzung des günstigen Überschussstromes wieder zunichtemacht.

Andere Alternativen sind die Umwandlung von Strom in Wasserstoff, der dann chemisch CO₂ in Methan umwandelt. Das Gas dient dann als Energiespeicher. Auch hierfür sind erhebliche Investitionen in die chemische Umwandlung zu tätigen. Außerdem besitzt Methan eine geringe Energiedichte und ist nicht gut transportierbar, da es als Gas vorliegt und entweder einer Pipeline oder einer teuren Verflüssigung bedarf. Eine höhere Dichte kann durch die chemische Umwandlung in Flüssigkeiten erzielt werden. Diese Verfahren enden jedoch entweder in relativ niederwertigen Stoffmischungen oder in Methanol, das wegen seines hohen Sauerstoffanteils auch nicht gut als Energieträger geeignet ist.

Für Kraftwerke, die auf Brennstoffen basieren, besteht neben der Möglichkeit, den Strom selber zu wandeln, auch die Option, die Energie des Brennstoffs vor der Stromerzeugung umzulenken, und diese Energie zur Wärmeerzeugung und anschließendes Heizen zu nutzen.

Solch ein Verfahren wird bisher insbesondere dort eingesetzt, wo die Anlieferung des Brennstoffs kontinuierlich erfolgt und nicht gedrosselt werden kann, wie zum Beispiel bei Kraftwerken, die ihre Energie aus den Abgasströmen industrieller Fertigungsanlagen, wie beispielsweise Stahlwerken, beziehen.

Dieses Verfahren hat den Nachteil, dass Wärme als Energieträger hohe Verluste durch mangelnde Isolierung erfährt und entsprechend einer zeitnahen Abnahme bedarf. Ebenso ist Wärme schlecht transportrietbar, so dass der Wärmeabnehmer in unmittelbarer Nähe des Kraftwerks angesiedelt sein muss.

US 2011/0067376, US 2010/0132257, WO 2007/123510, WO 2012/151545, WO 2009/154788, WO2012/174313, WO 2008/028055, WO 2012026833 und "carbon dioxide: capturing and utilization", A. Kargari et al Greenhouse Gases - Capturing, Utilization and Reduction; pages 1 - 30; 2012 offenbaren ein Verfahren und eine Vorrichtung zur Verwertung von Gasen enthaltend CO und/oder CO2.

Aufgabe der vorliegenden Erfindung ist es, für auf Brennstoff basierende Kraftwerke eine Möglichkeit zu schaffen, Überschussenergien zu speichern.

### Beschreibung der Erfindung

Überraschenderweise wurde gefunden, dass das im Folgenden beschriebene Verfahren die der Erfindung gestellte Aufgabe zu lösen vermag.

Gegenstand der vorliegenden Erfindung ist daher ein Verfahren zur Verwertung von Gasen enthaltend CO und/oder CO₂ wie im Anspruch 1 offenbart.

Ein Vorteil der vorliegenden Erfindung ist es, dass die Energiespeicherung der Überschussenergie vor der Verstromung erfolgt, somit ein Umwandlungsschritt weniger erfolgt und damit ein höherer Wirkungsgrad erzielt werden kann.

Ein weiterer Vorteil der vorliegenden Erfindung ist es, dass die Energiespeicherung der Überschussenergie bei Bedarf ausgesetzt werden kann und somit zeitlich diskontinuierlich durchgeführt werden kann.

Noch ein Vorteil der vorliegenden Erfindung ist es, dass die Energiespeicherung der Überschussenergie keiner nennenswerten Anlaufzeit bedarf, sondern sofort große Überschussenergiemengen nach deren Auftreten verwerten kann.

Ein weiterer Vorteil der vorliegenden Erfindung ist es, dass die Energiespeicherung der Überschussenergie geringen Raum- und Flächenbedarf aufweist.

Noch ein Vorteil der vorliegenden Erfindung ist es, dass der Energiespeicher der Überschussenergie eine hohe Energiedichte aufweist, und somit ein Transport der Energie bedeutend erleichtert wird.

Ein weiterer Vorteil der vorliegenden Erfindung ist es, dass die Energiespeicherung mit relativ geringen Investitionen durchgeführt werden kann, da keine Steriltechnik für die Fermentation notwendig ist

Noch ein Vorteil der vorliegenden Erfindung ist es, dass der Energiespeicher der Überschussenergie flüssig und somit leicht zu transportieren ist.

Ein weiterer Vorteil der vorliegenden Erfindung ist es, dass die Energiespeicher in ihrer Dimensionierung frei skalierbar sind.

Noch ein Vorteil der vorliegenden Erfindung ist es, dass der Energiespeicher mit stark schwankendem Energiefluss umgehen kann und somit einen idealen Puffer darstellt.

Ein erfindungsgemäß bevorzugtes Verfahren ist dadurch gekennzeichnet, dass Verfahrensschritt B) durchgeführt wird, während Verfahrensschritt C) durchgeführt wird.

Dies bedeutet, das gleichzeitig ein Teil des Gasstroms des Gases enthaltend CO und und/oder CO₂ zur Stromgewinnung genutzt wird, während ein anderer Teil zur Herstellung der organischen Substanz genutzt wird.

Die Größe der jeweiligen Gasströme kann dabei laufend variiert und angeglichen werden, bevorzugt in dem Maße wie es die Menge an Überschussenergie erfordert.

Im Extremfall kann gar temporär der gesamte Gasstrom zur Herstellung von organischer Substanz genutzt werden, was einem bevorzugten erfindungsgemäßem Verfahren entspricht, das dadurch gekennzeichnet ist, dass Verfahrensschritt B) nicht durchgeführt wird, während Verfahrensschritt C) durchgeführt wird.

Auf der anderen Seite kann auch bei einem hohen Strombedarf der gesamte Gasstrom zur Umwandlung in elektrische Energie genutzt werden, was einem bevorzugten erfindungsgemäßem Verfahren entspricht, das dadurch gekennzeichnet ist, dass Verfahrensschritt C) nicht durchgeführt wird, während Verfahrensschritt B) durchgeführt wird.

Die Verfahrensschritte B) und C) können in dem erfindungsgemäßen Verfahren wiederholt werden, bevorzugt mehrfach wiederholt werden, was einem bevorzugten erfindungsgemäßem Verfahren entspricht, dass dadurch gekennzeichnet, dass Verfahrensschritt D) durchgeführt wird.

Es ist erfindungsgemäß vorteilhaft ist, wenn das Gas enthaltend CO und/oder CO₂ ein reduzierendes Agens, bevorzugt Wasserstoff, enthält.

Dies hat den technischen Effekt, dass in Verfahrensschritt C) die benötigten Redoxäquivalente für das biotechnologische Verfahren bereits mit eingetragen werden. Das Gas enthaltend CO und/oder CO₂ ist Gichtgas aus einem Hochofen in der Stahlerzeugung.

Dies hat den technischen Effekt, dass Verfahrensschritt C) mit hoher Ausbeute betrieben werden kann, da dieser Gasstrom ein ideales Verhältnis CO, CO₂ und Wasserstoff aufweist.

In einem erfindungsgemäß bevorzugten Verfahren umfasst die Stromerzeugung in Verfahrensschritt B) mittels eines Gasturbinen- und/oder Dampfturbinenprozesses. Ein erfindungsgemäß bevorzugtes Verfahren ist dadurch gekennzeichnet, dass in Verfahrensschritt C) die organische Substanz ausgewählt ist aus organischen Substanzen umfassend mindestens drei, insbesondere mindestens vier Kohlenstoffatome, bevorzugt 3 bis 26, insbesondere 4 bis 20 Kohlenstoffatome, welche insbesondere bei 25 °C und 1 bar Druck flüssig sind.

Dies hat den technischen Vorteil, dass die Energiedichte in der organischen Substanz hoch ist und somit mehr Überschussenergie in einfach transportierbarer Form vorliegt.

Insbesondere bevorzugt ist die organische Substanz in Verfahrensschritt C) ausgewählt ist aus der Gruppe 1-Butanol,Isobutanol, Butandiol, Propan-2-ol, Aceton, 1-Propen, Buten, Isobuttersäure, 2 Hydroxyisobuttersäure, 2 Hydroxyisobuttersäure-Methylester, geradkettige und verzweigte Alkansäuren, welche gegebenenfalls mindestens eine Doppelbindung enthalten können, und deren Derivaten wie beispielsweise Buttersäure, Hexansäure und deren Ester, sowie die entsprechenden Alkanole.

Unter dem Begriff "Derivate der Alkansäuren" werden insbesondere die reduzierten Formen der Alkansäure, Aldehyd und Alkohol, die Alkansäureester, die omega-hydroxylierten Alkansäuren, die omega-aminierten Alkansäuren, die Alkansäureamide und die Disäuren und Diamine verstanden.

Das Verfahren ist dadurch gekennzeichnet, dass in Verfahrensschritt C) acetogene Bakterien eingesetzt werden.

Der Einsatz von acetogenen Bakterien hat den technischen Effekt, dass der Gasstrom für Verfahrensschritt C) temporär auf ein Minimum reduziert werden kann, ja sogar komplett unterbrochen werden kann. Diese Art von Bakterien, die es in der Natur gewohnt sind, unter widrigsten Verhältnissen zu überleben, können lange Zeit im Fermenter ohne besondere Fürsorge und Nahrung verweilen. Zusätzlich bewirkt der Einsatz von acteogenen Bakterien den technischen Effekt, dass Entstehende Überschussenergie sofort verwertet werden kann, da die Bakterien bei Wiedereinsetzen des Gasstroms unverzüglich ihren Stoffwechsel wieder aufnehmen und das Gas zu organischer Substanz umsetzen.

Unter dem Begriff "acetogenes Bakterium" wird ein Bakterium verstanden, welches in der Lage ist, den Wood-Ljungdahl Stoffwechselweg durchzuführen und somit in der Lage ist, CO und CO₂ und Wasserstoff zu Acetat umzusetzen.

Der Begriff "acetogenes Bakterium" umfasst auch solche Bakterien, die ursprünglich als Wildtyp keinen Wood-Ljungdahl Stoffwechselweg aufweisen, sondern diesen erst aufgrund gentechnischer Modifikation aufweisen. Solche Bakterien können beispielsweise *E. coli* Zellen sein.

Bevorzugt in Verfahrensschritt C) eingesetzte acetogene Bakterien weisen verglichen zu ihrem Wildtyp eine erhöhte Enzymaktivität eines Wood-Ljungdahl Stoffwechselwegenzyms aufgrund von gentechnischer Modifikation auf. Bevorzugte Wood-Ljungdahl Stoffwechselwegenzyme in diesem Zusammenahng sind ausgewählt aus CO-Dehydrogenasen und Acetyl-CoA Synthetasen.

Acetogene Baktereien, welche CO₂ und/oder CO umsetzen, sowie geeignete Verfahren und Verfahrensbedingungen, die in Verfahrensschritt C) zum Einsatz kommen sind seit Langem bekannt. Solche Prozesse werden beispielsweise
in der WO9800558, WO2000014052, WO2010115054
in Demler et al. Reaction engineering analysis of hydrogenotrophic production of acetic acid by Acetobacterium woodii. Biotechnol Bioeng. 2011 Feb;108(2):470-4,
in Younesia et al. Ethanol and acetate production from synthesisgas via fermentation processes using anaerobic bacterium, Clostridium ljungdahlii. Biochemical Engineering Journal, Volume 27, Issue 2, Pages 110-119,
in Morinaga et al. The production of acetic acid from carbon dioxide and hydrogen by an anaerobic bacterium. Journal of Biotechnology, Volume 14, Issue 2, Pages 187-194,
in Li Production of acetic acid from synthesis gas with mixed acetogenic microorganisms, ISSN 0493644938,
in Schmidt et al. Production of acetic acid from hydrogen and carbon dioxide by clostridium species ATCC 2979. Chemical Engineering Communications, 45:1-6, 61-73,
in Sim et al. Optimization of acetic acid production from synthesis gas by chemolithotrophic bacterium - Clostridium aceticum using a statistical approach. Bioresour Technol. 2008 May;99(8):2724-35,
in Vega et al. Study of gaseus substrate fermentations CO conversion to acetate 1 Batch culture bzw. 2 continous culture. Biotechnology and Bioengineering Volume 34, Issue 6, pages 774, bzw. 785, September 1989,
in Cotter et al. Ethanol and acetate production by Clostridium ljungdahlii and Clostridium autoethanogenum using resting cells. Bioprocess and Biosystems Engineering (2009), 32(3), 369-380 und
in Andreesen et al. Fermentation of glucose, fructose, and xylose by Clostridium thermoaceticum. Effect of metals on growth yield, enzymes, and the synthesis of acetate from carbon dioxide. Journal of Bacteriology (1973), 114(2), 743-51 beschrieben.

Dem Fachmann bietet sich hieraus eine große Anzahl von ausführbaren Möglichkeiten zur Auslegung des Verfahrensschrittes C), die allesamt gut funktionieren.

Besonders bevorzugt werden in Verfahrensschritt C) acetogene Bakterien eingesetzt ausgewählt aus der Gruppe umfassend *Clostridium autothenogenum DSMZ 19630, Clostridium ragsdahlei ATCC no. BAA-622, Clostridium autoethanogenum, Moorella sp HUC22-1, Moorella thermoaceticum, Moorella thermoautotrophica, Rumicoccus productus, Acetoanaerobum, Oxobacter pfennigii, Methanosarcina barkeri, Methanosarcina acetivorans, Carboxydothermus, Desulfotomaculum kutznetsovii, Pyrococcus, Peptostreptococcus, Butyribacterium methylotrophicum ATCC 33266*, *Clostridium formicoaceticum, Clostridium butyricum, Laktobacillus delbrukii, Propionibacterium acidoprprionici, Proprionispera arboris, Anaerobierspirillum succiniproducens, Bacterioides amylophilus, Becterioides ruminicola, Thermoanaerobacter kivui, Acetobacterium woodii, Acetoanaerobium notera, Clostridium aceticum, Butyribacterium methylotrophicum, Moorella thermoacetica, Eubacterium limosum, Peptostreptococcus productus, Clostridium ljungdahlii, Clostridium ATCC 29797* und *Clostridium carboxidivorans,* insbesondere ATCC BAA-624. Ein besonders geeignetes Bakterium ist *Clostridium carboxidivorans,* insbesondere solche Stämme wie "P7" und "P11". Solche Zellen sind beispielsweise in US 2007/0275447 und US 2008/0057554 beschrieben. Ein weiteres, besonders geeignetes Bakterium ist *Clostridium ljungdahlii,* insbesondere Stämme ausgewählt aus der Gruppe umfassend *Clostridium ljungdahlii* PETC, *Clostridium ljungdahlii* ERI2, *Clostridium ljungdahlii* C0I und *Clostridium ljungdahlii* O-52, diese werden in der WO 98/00558 und WO 00/68407 beschrieben, sowie ATCC 49587, ATCC 55988 und ATCC 55989.

In einer alternativ bevorzugten Ausführungsform des erfindungsgemäßen Verfahrens wird in Verfahrensschritt C) Ethanol gebildet und als Mikroorganismus *Alkalibaculum bacchi* ATCC BAA-1772, *Moorella sp.* HUC22-1, *Clostridium ljungdahlii, Clostridium ragsdahlei,* oder *Clostridium autoethanogenum* eingesetzt. Entsprechende Anweiseungen zur Durchführung des Verfahrensschrittes A) erhält man aus beispielsweise Saxena et al. *Effect of trace metals on ethanol production from synthesis gas by the ethanologenic acetogen Clostridium ragsdalei.*

Journal of Industrial Microbiology & Biotechnology Volume 38, Number 4 (2011), 513-521, Younesi et al. Ethanol and acetate production from synthesis gas via fermentation processes using anaerobic bacteriumClostridium ljungdahlii. Biochemical Engineering Journal Volume 27, Issue 2, 15 December 2005, Pages 110-119,
Sakai et al. Ethanol production from H2 and CO2 by a newly isolated thermophilic bacterium, Moorella sp. HUC22-1. Biotechnology Letters Volume 26, Number 20 (2004), 1607-1612 und Abrini et al. Clostridium autoethanogenum, sp. nov., an anaerobic bacterium that produces ethanol from carbon monoxide. Archives of Microbiology Volume 161, Number 4 (1994), 345-351.

In einer alternativ bevorzugten Ausführungsform des erfindungsgemäßen Verfahrens wird in Verfahrensschritt C) Ethyl-Acetat gebildet und ein acetogenes Bakterium eingesetzt. Eine Anleitung zur Durchführung des Verfahrensschrittes C) dieser alternativ bevorzugten Ausführungsform ist in der WO2012162321 beschrieben.

In einer alternativ bevorzugten Ausführungsform des erfindungsgemäßen Verfahrens wird in Verfahrensschritt C) Butanol gebildet und ein acetogenes Bakterium eingesetzt. Eine Anleitung zur Durchführung des Verfahrensschrittes C) dieser alternativ bevorzugten Ausführungsform ist in der US20110236941 beschrieben.

In einer alternativ bevorzugten Ausführungsform des erfindungsgemäßen Verfahrens wird in Verfahrensschritt C) Hexanol gebildet und ein acetogenes Bakterium eingesetzt. Eine Anleitung zur Durchführung des Verfahrensschrittes C) dieser alternativ bevorzugten Ausführungsform ist in der US20100151543 beschrieben.

In einer alternativ bevorzugten Ausführungsform des erfindungsgemäßen Verfahrens wird in Verfahrensschritt C) 2,3-Butanediol gebildet und ein acetogenes Bakterium eingesetzt. Eine Anleitung zur Durchführung des Verfahrensschrittes C) dieser alternativ bevorzugten Ausführungsform ist in der US20120252082 und WO2012131627 beschrieben.

In einer alternativ bevorzugten Ausführungsform des erfindungsgemäßen Verfahrens wird in Verfahrensschritt C) Isopropanol gebildet und ein acetogenes Bakterium eingesetzt.

Eine Anleitung zur Durchführung des Verfahrensschrittes C) dieser alternativ bevorzugten Ausführungsform ist in der US20120252083 beschrieben.

In einer alternativ bevorzugten Ausführungsform des erfindungsgemäßen Verfahrens wird in Verfahrensschritt C) 2-Hydroxyisobuttersäure gebildet und ein acetogenes Bakterium eingesetzt.

Eine Anleitung zur Durchführung des Verfahrensschrittes C) dieser alternativ bevorzugten Ausführungsform ist in der EP12173010 beschrieben.

Verfahrensschritt C) wird unter Einsatz von acetogenen Bakterien bevorzugt unter anaeroben Bedingungen ausgeführt.

Ein weiterer Gegenstand der vorliegenden Erfindung bildet eine Vorrichtung wie im Anspruch 9 offenbart.

Die Verstromungseinrichtung der erfindungsgemäßen Vorrichtung umfasst bevorzugt einen von mindestens einer Turbine angetriebenen Generator.

In diesem Zusammenhang handelt es sich bei der Turbine bevorzugt um eine Gasturbine, die vollständig oder unter Zumischung weiterer Brennstoffe mit dem Gasstrom betreibbar ist.

Die erfindungsgemäße Vorrichtung ist bevorzugt dadurch gekennzeichnet, dass die Mittel zum wahlweisen Beaufschlagen des Fermenters und/oder der Verstromungseinrichtug mit Gasstrom diese Apparate verbindende Leitungen und Stellorgane umfassen.

In diesem Zusammenhang ist es bevorzugt, dass die Stellorgane und die Leitungen dafür eingerichtet sind, den Fermenter und die Verstromungseinrichtung parallel und/oder seriell und/oder einzeln mit dem Gasstrom zu beaufschlagen.

Insbesondere bevorzugt ist die erfindungsgemäße Vorrichtung dadurch gekennzeichnet, dass sämtliche Bestandteile der Vorrichtung in einen Verbundstandort eingegliedert sind.

Ein weiterer Gegenstand der vorliegenden Erfindung bildet die Verwendung der erfindungsgemäßen Vorrichtung zur Durchführung des erfindungsgemäßen Verfahrens.

### Beispiele

Die vorliegende Erfindung soll nun anhand eines Ausführungsbeispiels näher erläutert werden. Hierfür zeigt:

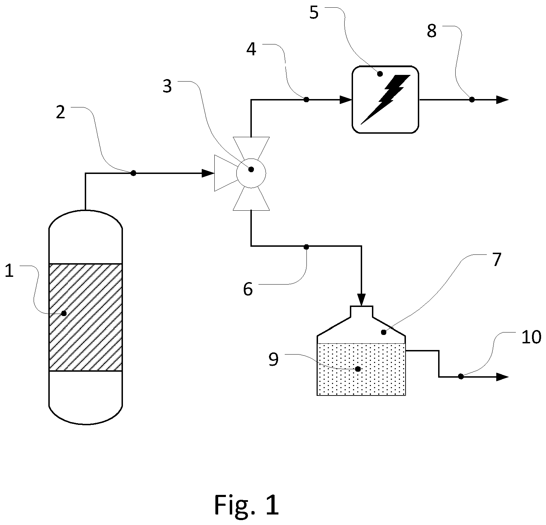

Figur 1: erfindungsgemäße Vorrichtung zur Durchführung des Verfahrens (schematisch).

Figur 1 zeigt den schematischen Aufbau einer erfindungsgemäßen Vorrichtung zur Durchführung des Verfahrens.

Eine Gasquelle 1 in Gestalt eines konventionellen Hochofens für die Stahlerzeugung liefert an seinem Kopf kontinuierlich Gichtgas, was über eine entsprechende Gasleitung 2 abgezogen wird. Das bei der Stahlerzeugung entstehende Gichtgas ist ein brennbares Kuppelgas, mit einem Stickstoffgehalt von etwa 45 - 60 % und einem Anteil von CO im Bereich von 20 - 30 %. Des Weiteren enthält das Gichtgas noch ca. 20 - 25 % CO₂ und 2 - 4 % H₂.

Das Gichtgas wird zu einem Stellorgan 3 geleitet. Dabei handelt es sich um ein an sich bekanntes Ventil, welches es gestattet, das von der Gasquelle 1 anströmende Gas wahlweise über eine Gasleitung 4 in Richtung einer Verstromungseinrichtung 5 und/oder über eine Gasleitung 6 in Richtung eines Fermenters 7 zu leiten. Das Stellorgan 3 ermöglicht dabei entweder den Gasstrom vollständig und allein in die Verstromungseinrichtung 5 zu leiten oder vollständig und allein in den Fermenter 7. Zusätzlich kann das Stellorgan 3 Zwischenstellungen einnehmen, die eine gleichzeitige Beaufschlagung der Verstromungseinrichtung 5 und des Fermenters 7 mit dem Gasstrom ermöglichen und zwar zu gleichen Teilen oder zu unterschiedlichen Anteilen.

Das in die Verstromungseinrichtung 5 gelangte Gas wird darin über einen an sich bekannten herkömmlichen Gasturbinen- und/oder Dampfturbinen-Prozess in elektrische Energie umgesetzt, die als elektrischer Strom 8 aus der Verstromungseinrichtung 5 abgezogen wird. Sofern eine Dampfturbine eingesetzt wird kann diese ausschließlich mit dem Gas aus der Gasquelle 1 betrieben werden oder unter Zugabe von externen Brennstoffen. Sofern ein Dampfturbinen-Prozess eingesetzt wird, wird der Kessel zur Dampferzeugung auch wahlweise mit dem Gas aus der Gasquelle 1 oder zusätzlich unter Zuhilfenahme von externen Brennstoffen beheizt. Es ist auch möglich, innerhalb der Verstromungseinrichtung 5 einen Gasturbinen-Prozess mit einem Dampfturbinen-Prozess zu koppeln. Die hier geschilderten Technologien zur Verstromung von Gas sind im Stand der Technik hinlänglich bekannt und bedürfen hier keiner näheren Schilderung.

Die Anteile des aus der Gasquelle 1 stammenden Gases, die über die Gasleitung 6 in Richtung des Fermenters 7 geleitet werden, werden darin von Bakterien 9 in eine organische Substanz 10 umgesetzt, die aus dem Fermenter 7 abgezogen wird.

Bei den Bakterien 9 handelt es sich um aceotogene Bakterien . Geeignete Bakterien und fermentative Verfahren zur Umsetzung von CO und/oder CO₂ enthaltenen Gasen in organischen Substanzen sind aus dem oben zitierten Stand der Technik hinlänglich bekannt und brauchen deswegen nicht näher geschildert werden.

### Bezugszeichenliste

1 Gasquelle / Hochofen
2 Gasleitung für Gichtgas
3 Stellorgan
4 Gasleitung in Richtung Verstromungseinrichtung
5 Verstromungseinrichtung
6 Gasleitung in Richtung Fermenter
7 Fermenter
8 elektrischer Strom
9 Bakterien
10 organische Substanz

## Patentansprüche

1. Verfahren zur Verwertung von Gasen enthaltend CO und/oder CO₂ umfassend die Verfahrensschritte:
A) Bereitstellen eines Gasstroms des Gases enthaltend CO und/oder CO₂,
B) Umwandlung mindestens eines Teils des Gasstroms in elektrische Energie,
C) Umsetzen mindestens eines Teils des Gasstroms zu mindestens einer organischen Substanz in einem biotechnologischen, fermentativen Verfahren und gegebenenfalls
D) Wiederholen der Verfahrensschritte B) und C)
**dadurch gekennzeichnet,**
**dass** in Verfahrensschritt C) acetogene Bakterien eingesetzt werden und
**dass** Verfahrensschritt A) den Einsatz von Gichtgas aus einem Hochofen in der Stahlerzeugung umfasst.

2. Verfahren gemäß Anspruch 1,
**dadurch gekennzeichnet,**
**dass** Verfahrensschritt B) durchgeführt wird, während Verfahrensschritt C) durchgeführt wird.

3. Verfahren gemäß Anspruch 1,
**dadurch gekennzeichnet,**
**dass** Verfahrensschritt B) nicht durchgeführt wird, während Verfahrensschritt C) durchgeführt wird.

4. Verfahren gemäß Anspruch 3,
**dadurch gekennzeichnet,**
**dass** Verfahrensschritt D) durchgeführt wird.

5. Verfahren gemäß mindestens einem der vorherigen Ansprüche,
**dadurch gekennzeichnet,**
**dass** Verfahrensschritt B) Stromerzeugung mittels eines Gasturbinen- und/oder Dampfturbinenprozesses umfasst.

6. Verfahren gemäß mindestens einem der vorherigen Ansprüche,
**dadurch gekennzeichnet,**
**dass** in Verfahrensschritt C) die organische Substanz ausgewählt ist aus organischen Substanzen umfassend mindestens drei, insbesondere mindestens vier Kohlenstoffatome.

7. Verfahren gemäß mindestens einem der vorherigen Ansprüche,
**dadurch gekennzeichnet,**
**dass** in Verfahrensschritt C) die organische Substanz ausgewählt ist aus der Gruppe 1-Butanol,lsobutanol, Butandiol, Propan-2-ol, Aceton, 1-Propen, Buten, Isobuttersäure, 2 Hydroxyisobuttersäure, 2 Hydroxyisobuttersäure-Methylester, geradkettige und verzweigte Alkansäuren, welche gegebenenfalls mindestens eine Doppelbindung enthalten können, und deren Derivaten wie beispielsweise Buttersäure, Hexansäure und deren Ester, sowie die entsprechenden Alkanole.

8. Verfahren gemäß mindestens einem der vorherigen Ansprüche,
**dadurch gekennzeichnet,**
**dass** in Verfahrensschritt C) acetogene Bakterien ausgewählt aus *Clostridium autothenogenum DSMZ 19630, Clostridium ragsdahlei ATCC no. BAA-622, Clostridium autoethanogenum, Moorella sp HUC22-1, Moorella thermoaceticum, Moorella thermoautotrophica, Rumicoccus productus, Acetoanaerobum, Oxobacter pfennigii, Methanosarcina barkeri, Methanosarcina acetivorans, Carboxydothermus, Desulfotomaculum kutznetsovii, Pyrococcus, Peptostreptococcus, Butyribacterium methylotrophicum ATCC* 33266, *Clostridium formicoaceticum, Clostridium butyricum, Laktobacillus delbrukii, Propionibacterium acidoprprionici, Proprionispera arboris, Anaerobierspirillum succiniproducens, Bacterioides amylophilus, Becterioides ruminicola, Thermoanaerobacter kivui, Acetobacterium woodii, Acetoanaerobium notera, Clostridium aceticum, Butyribacterium methylotrophicum, Moorella thermoacetica, Eubacterium limosum, Peptostreptococcus productus, Clostridium ljungdahlii, Clostridium ATCC 29797* und *Clostridium carboxidivorans* eingesetzt werden.

9. Vorrichtung zur Durchführung eines Verfahrens nach einem der vorhergehenden Ansprüche, umfassend:
a) eine Gasquelle zur kontinuierlichen Bereitstellung eines CO und/oder CO₂ enthaltenden Gasstroms;
b) eine Verstromungseinrichtung zum Umwandeln von aus der Gasquelle stammenden Gases in elektrische Energie;
c) einen Fermenter zum Umsetzen von aus der Gasquelle stammenden Gases in mindestens eine organische Substanz;
d) und Mittel zum wahlweisen Beaufschlagen der Verstromungsanlage und/oder des Fermenters mit dem Gasstrom aus der Gasquelle,
**dadurch gekennzeichnet, dass** der Fermenter acetogene Bakterien enthält, und dass es sich bei der Gasquelle um einen in der Stahlerzeugung eingesetzten Hochofen handelt, der als Gasstrom kontinuierlich Gichtgas liefert.

10. Vorrichtung nach Anspruch 9,
**dadurch gekennzeichnet,**
**dass** die Verstromungseinrichtung einen von mindestens einer Turbine angetriebenen Generator umfasst.

11. Vorrichtung nach Anspruch 10,
**dadurch gekennzeichnet,**
**dass** es sich bei der Turbine um eine Gasturbine handelt, die vollständig oder unter Zumischung weiterer Brennstoffe mit dem Gasstrom betreibbar ist.

12. Vorrichtung nach einem der Ansprüche 9 bis 11,
**dadurch gekennzeichnet,**
**dass** die Mittel zum wahlweisen Beaufschlagen des Fermenters und/oder der Verstromungseinrichtung mit Gasstrom diese Apparate verbindende Leitungen und Stellorgane umfassen.

13. Vorrichtung nach Anspruch 12,
**dadurch gekennzeichnet,**
**dass** die Stellorgane und die Leitungen dafür eingerichtet sind, den Fermenter und die Verstromungseinrichtung parallel und/oder seriell und/oder einzeln mit dem Gasstrom zu beaufschlagen.

14. Vorrichtung nach einem der Ansprüche 9 bis 13,
**dadurch gekennzeichnet,**
**dass** sämtliche Bestandteile der Vorrichtung in einen Verbundstandort eingegliedert sind.

15. Verfahren nach einem der Ansprüche 1 bis 8 unter Verwendung einer Vorrichtung nach einem der Ansprüche 9 bis 14.

## Claims

1. Method for utilizing gases containing CO and/or CO₂ comprising the method steps of:
A) providing a gas stream of gas containing CO and/or CO₂,
B) converting at least a part of the gas stream into electrical energy,
C) converting at least a part of the gas stream to at least one organic substance in a biotechnological fermentation process and optionally
D) repeating method steps B) and C) **characterized in that**
acetogenic bacteria are used in method step C) and **in that** method step A) includes the use of blast furnace gas from a blast furnace in steel-making.

2. Method according to Claim 1,
**characterized in that**
method step B) is carried out while method step C) is carried out.

3. Method according to Claim 1,
**characterized in that**
method step B) is not carried out while method step C) is carried out.

4. Method according to Claim 3,
**characterized in that**
method step D) is carried out.

5. Method according to at least one of the preceding claims,
**characterized in that**
method step B) includes generating electricity by means of a gas turbine and/or steam turbine process.

6. Method according to at least one of the preceding claims,
**characterized in that**
the organic substance in method step C) is selected from organic substances comprising at least three, particularly at least four carbon atoms.

7. Method according to at least one of the preceding claims,
**characterized in that**
the organic substance in method step C) is selected from the group of 1-butanol, isobutanol, butanediol, propan-2-ol, acetone, 1-propene, butene, isobutyric acid, 2-hydroxyisobutyric acid, methyl 2-hydroxyisobutyrate, straight-chain and branched alkanoic acids, which may optionally comprise at least one double bond, and derivatives thereof, such as butyric acid, hexanoic acid and esters thereof, and also the corresponding alkanols.

8. Method according to at least one of the preceding claims,
**characterized in that**
acetogenic bacteria used in method step C) are selected from *Clostridium autothenogenum DSMZ 19630, Clostridium ragsdahlei ATCC no. BAA-622, Clostridium autoethanogenum, Moorella sp HUC22-1, Moorella thermoaceticum, Moorella thermoautotrophica, Rumicoccus productus, Acetoanaerobum, Oxobacter pfennigii, Methanosarcina barkeri, Methanosarcina acetivorans, Carboxydothermus, Desulfotomaculum kutznetsovii, Pyrococcus, Peptostreptococcus, Butyribacterium methylotrophicum ATCC 33266, Clostridium formicoaceticum, Clostridium butyricum, Laktobacillus delbrukii, Propionibacterium acidoprprionici, Proprionispera arboris, Anaerobierspirillum succiniproducens, Bacterioides amylophilus, Becterioides ruminicola, Thermoanaerobacter kivui, Acetobacterium woodii, Acetoanaerobium notera, Clostridium aceticum, Butyribacterium methylotrophicum, Moorella thermoacetica, Eubacterium limosum, Peptostreptococcus productus, Clostridium ljungdahlii, Clostridium ATCC 29797* and *Clostridium carboxidivorans.*

9. Apparatus for carrying out a method according to any of the preceding claims, comprising:
a) a gas source for continuously providing a gas stream containing CO and/or CO₂;
b) a power-generating device for converting gases originating from the gas source into electrical energy;
c) a fermenter for converting gases originating from the gas source into at least one organic substance;
d) and means for selectively feeding the gas stream from the gas source to the power-generating plant and/or to the fermenter,
**characterized in that** the fermenter comprises acetogenic bacteria, and that the gas source is a blast furnace used in steel-making which continuously provides blast furnace gas as gas stream.

10. Apparatus according to Claim 9,
**characterized in that**
the power-generating device includes one generator driven by at least one turbine.

11. Apparatus according to Claim 10,
**characterized in that**
the turbine is a gas turbine which can be operated entirely by the gas stream or by mixing with other fuels.

12. Apparatus according to any of Claims 9 to 11,
**characterized in that**
the means for selectively feeding the gas stream to the fermenter and/or to the power-generating device include lines and control elements connecting these apparatus.

13. Apparatus according to Claim 12,
**characterized in that**
the control elements and the lines are adapted to feed the fermenter and the power-generating device with the gas stream in a parallel and/or serial and/or individual manner.

14. Apparatus according to Claims 9 to 13,
**characterized in that**
all the components of the apparatus are integrated in one Verbund site.

15. Method according to any of Claims 1 to 8 for use of an apparatus according to any of Claims 9 to 14.

## Revendications

1. Procédé de valorisation de gaz contenant du CO et/ou du CO₂, comprenant les étapes de procédé suivantes :
A) la mise à disposition d'un courant gazeux du gaz contenant du CO et/ou du CO₂,
B) la transformation d'au moins une partie du courant gazeux en énergie électrique,
C) la conversion d'au moins une partie du courant gazeux en au moins une substance organique dans un procédé fermentatif biotechnologique et éventuellement
D) la répétition des étapes de procédé B) et C),
**caractérisé en ce que**
dans l'étape de procédé C), des bactéries acétogènes sont utilisées et
**en ce que** l'étape de procédé A) comprend l'utilisation de gaz de haut fourneau issu d'un haut fourneau dans la production d'acier.

2. Procédé selon la revendication 1,
**caractérisé en ce que**
l'étape de procédé B) est réalisée pendant que l'étape de procédé C) est réalisée.

3. Procédé selon la revendication 1,
**caractérisé en ce que**
l'étape de procédé B) n'est pas réalisée pendant que l'étape de procédé C) est réalisée.

4. Procédé selon la revendication 3,
**caractérisé en ce que**
l'étape de procédé D) est réalisée.

5. Procédé selon au moins l'une quelconque des revendications précédentes,
**caractérisé en ce que**
l'étape de procédé B) comprend une production électrique au moyen d'un processus à turbine à gaz et/ou à turbine à vapeur.

6. Procédé selon au moins l'une quelconque des revendications précédentes,
**caractérisé en ce que**
dans l'étape de procédé C), la substance organique est choisie parmi les substances organiques comprenant au moins trois, notamment au moins quatre, atomes de carbone.

7. Procédé selon au moins l'une quelconque des revendications précédentes,
**caractérisé en ce que**
dans l'étape de procédé C), la substance organique est choisie dans le groupe constitué par le 1-butanol, l'isobutanol, le butanediol, le propan-2-ol, l'acétone, le 1-propène, le butène, l'acide isobutyrique, l'acide 2-hydroxyisobutyrique, l'ester méthylique de l'acide 2-hydroxyisobutyrique, les acides alcanoïques linéaires et ramifiés, qui peuvent éventuellement contenir au moins une double liaison, et leurs dérivés tels que par exemple l'acide butyrique, l'acide hexanoïque et leurs esters, ainsi que les alcanols correspondants.

8. Procédé selon au moins l'une quelconque des revendications précédentes,
**caractérisé en ce que**
dans l'étape de procédé C), des batéries acétogènes choisies parmi *Clostridium autothenogenum* DSMZ 19630, *Clostridium ragsdahlei ATCC no. BAA-622, Clostridium autoethanogenum, Moorella sp HUC22-1, Moorella thermoaceticum, Moorella thermoautotrophica, Rumicoccus productus, Acetoanaerobum, Oxobacter pfennigii, Methanosarcina barkeri, Methanosarcina acetivorans, Carboxydothermus, Desulfotomaculum kutznetsovii, Pyrococcus, Peptostreptococcus, Butyribacterium methylotrophicum ATCC 33266, Clostridium formicoaceticum, Clostridium butyricum, Laktobacillus delbrukii, Propionibacterium acidoprprionici, Proprionispera arboris, Anaerobierspirillum succiniproducens, Bacterioides amylophilus, Becterioides ruminicola, Thermoanaerobacter kivui, Acetobacterium woodii, Acetoanaerobium notera, Clostridium aceticum, Butyribacterium methylotrophicum, Moorella thermoacetica, Eubacterium limosum, Peptostreptococcus productus, Clostridium ljungdahlii, Clostridium ATCC 29797* et *Clostridium carboxidivorans* sont utilisées.

9. Dispositif pour la réalisation d'un procédé selon l'une quelconque des revendications précédentes, comprenant :
a) une source de gaz pour la mise à disposition continue d'un courant gazeux contenant du CO et/ou du CO₂ ;
b) un équipement de production d'électricité pour la transformation de gaz provenant de la source de gaz en énergie électrique ;
c) un fermentateur pour la conversion de gaz provenant de la source de gaz en au moins une substance organique ;
d) et des moyens pour l'alimentation sélective de l'unité de production d'électricité et/ou du fermentateur avec le courant gazeux de la source de gaz,
**caractérisé en ce que** le fermentateur contient des bactéries acétogènes, et **en ce que** la source de gaz consiste en un haut fourneau utilisé dans la production d'acier, qui fournit du gaz de haut fourneau en continu en tant que courant gazeux.

10. Dispositif selon la revendication 9,
**caractérisé en ce que**
l'équipement de production d'électricité comprend un générateur entraîné par au moins une turbine.

11. Dispositif selon la revendication 10,
**caractérisé en ce que**
la turbine consiste en une turbine à gaz, qui peut être exploitée avec le courant gazeux en totalité ou avec mélange de combustibles supplémentaire.

12. Dispositif selon l'une quelconque des revendications 9 à 11,
**caractérisé en ce que**
les moyens pour l'alimentation sélective du fermentateur et/ou de l'équipement de production d'électricité avec le courant gazeux comprennent des conduites reliant ces appareils et des organes de réglage.

13. Dispositif selon la revendication 12,
**caractérisé en ce que**
les organes de réglages et les conduites sont conçus pour alimenter le fermentateur et l'équipement de production d'électricité en parallèle et/ou en série et/ou individuellement avec le courant gazeux.

14. Dispositif selon l'une quelconque des revendications 9 à 13,
**caractérisé en ce que**
tous les constituants du dispositif sont intégrés dans un site mixte.

15. Procédé selon l'une quelconque des revendications 1 à 8 utilisant un dispositif selon l'une quelconque des revendications 9 à 14.
